(19) Europäisches Patentamt
European Patent Office
Office européen des brevets

(11) Veröffentlichungsnummer: **0 290 861**
**B1**

(12) # EUROPÄISCHE PATENTSCHRIFT

(45) Veröffentlichungstag der Patentschrift: **04.07.90**

(21) Anmeldenummer: **88106701.1**

(22) Anmeldetag: **27.04.88**

(51) Int. Cl.[5]: **C07C 1/26,** C07C 17/00, C07B 35/06

(54) **Verfahren zur reduktiven Dehalogenierung von organischen Halogenverbindungen.**

(30) Priorität: **12.05.87 DE 3715751**

(43) Veröffentlichungstag der Anmeldung: **17.11.88 Patentblatt 88/46**

(45) Bekanntmachung des Hinweises auf die Patenterteilung: **04.07.90 Patentblatt 90/27**

(84) Benannte Vertragsstaaten: **AT BE CH DE FR GB IT LI NL**

(56) Entgegenhaltungen:
**DD-A- 235 630**
**DE-A- 3 510 033**
**DE-A- 3 510 034**

(73) Patentinhaber: **BASF Aktiengesellschaft, Carl-Bosch-Strasse 38, D-6700 Ludwigshafen(DE)**

(72) Erfinder: **Dockner, Toni, Dr., Grossgasse 6, D-6701 Meckenheim(DE)**
Erfinder: **Sauerwald, Manfred, Dr., Gebhardtstrasse 16, D-6701 Roedersheim-Gronau(DE)**
Erfinder: **Krug, Herbert, Mussbacher Strasse 49, D-6700 Ludwigshafen(DE)**
Erfinder: **Irgang, Matthias, Dr., Andreas-Hofer-Weg 41, D-6900 Heidelberg(DE)**

**EP 0 290 861 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur reduktiven Dehalogenierung von organischen Halogenverbindungen, bei denen mindestens ein Halogenatom kovalent am Kohlenstoff gebunden ist, durch Umsetzung mit Kohlenwasserstoffen in Gegenwart von Kohlenstoff bei erhöhter Temperatur unter Bildung von Halogenwasserstoffen.

Ein breit anwendbares und technisch leicht durchführbares Verfahren zur reduktiven Dehalogenierung von organischen Halogenverbindungen unter Bildung von Kohlen- bzw. Halogenwasserstoffen wird in der DE-A 3 510 033 und der DE-A 3 510 034 beschrieben. Danach werden aliphatische, cycloaliphatische, aromatische oder araliphatische Halogenverbindungen in der Flüssigphase bei Temperaturen von 100 bis 450°C oder in der Gasphase bei Temperaturen von 200 bis 600°C mit Kohlenwasserstoffen in Gegenwart von Kohlenstoff umgesetzt. Als Kohlenwasserstoffe werden z.B. bei Reaktionen in der Flüssigphase hochsiedende Mineralölfraktionen wie Vakuumrückstand, Heizöl S oder Technisches Weißöl oder bei Reaktionen in der Gasphase billige, niedrig siedende aliphatische Kohlenwasserstoffe wie Isobutan verwendet.

Nachteilig an diesen Verfahren ist die noch nicht ausreichende Ausnutzung des Wasserstoffgehalts der Kohlenwasserstoffe für die reduktive Dehalogenierung (s. Vergleichsbeispiele).

Aus der DD-A 235 630 ist ein Verfahren bekannt, nach dem 1,2-Dichlorpropan ohne Zusatz von Kohlenwasserstoffen in der Gasphase bei Temperaturen von 170 bis 450°C an Aktivkohle, die mit einer Suspension von Eisenoxiden und/oder Eisenoxidhydraten behandelt und anschließend bei 80 bis 200°C getrocknet worden ist, zu Propen und Chlorpropen gespalten wird. Das gasförmige Reaktionsgemisch über das keine Mengenangaben vorliegen, besteht jedoch nur zu 75,2 Vol.% aus Propen, der Rest ist cis/trans-1-Chlorpropen.

Der Erfindung lag nun die Aufgabe zugrunde, ein Dehalogenierungsverfahren zu finden, das sich durch hohe Umsätze, hohe Selektivität und bessere Ausnutzung des zugesetzten Kohlenwasserstoffs auszeichnet.

Demgemäß wurde ein Verfahren zur reduktiven Dehalogenierung von organischen Halogenverbindungen, bei denen mindestens ein Halogenatom kovalent am Kohlenstoff gebunden ist, durch Umsetzung mit Kohlenwasserstoffen in Gegenwart von Kohlenstoff bei erhöhter Temperatur unter Bildung von Halogenwasserstoffen gefunden, das dadurch gekennzeichnet ist, daß man die Dehalogenierung in Gegenwart einer Eisenverbindung als Cokatalysator bei Temperaturen von 100 bis 450°C durchführt.

Der für die Bildung von Halogenwasserstoff benötigte Wasserstoff entstammt dem Kohlenwasserstoff, aus dem neben wasserstoffärmeren Derivaten überwiegend Kohlenstoff gebildet wird.

Als Kohlenwasserstoffe können vorteilhaft hochsiedende Mineralöle verwendet werden, deren Siedetemperaturen höher liegen als die Reaktionstemperatur, die 100 bis 450, vorzugsweise 200 bis 400, insbesondere 250 bis 350°C beträgt. Solche Kohlenwasserstoffe sind z.B. Vakuumgasöl, Technisches Weißöl, Heizöl, Heizöl S, Vakuumrückstandsöl oder sonstige bei der Erdölfraktionierung anfallende hochsiedende Bestandteile.

Darüber hinaus können aber auch niedriger siedende Kohlenwasserstoffe wie Methan, Ethan, Acetylen, Propan, Propen, Butan, Buten, Pentan, Penten, Cyclopentan, Hexan, Hexen, Cyclohexan oder 1,2,3,4-Tetrahydronaphthalin bzw. niedriger siedende Kohlenwasserstoffgemische wie z.B. Heizöl L, Benzin, Leichtbenzin oder Flüssiggas eingesetzt werden.

Die Mengenverhältnisse von Kohlenwasserstoff zu Halogenverbindung liegen in der Regel bei 0,2 bis 5, insbesondere 0,5 bis 3.

Die Durchführung der Dehalogenierungsreaktion kann sowohl bei Normaldruck, als auch bei Über- bzw. Unterdruck erfolgen. Während bei Verwendung der hochsiedenden Kohlenwasserstoffe in der Regel Normaldruck am zweckmäßigsten ist, wird bei Verwendung der niedriger siedenden Kohlenwasserstoffe die Umsetzung am besten in der Gasphase oder unter Druck in der Flüssigphase durchgeführt.

Bei der Reaktion in flüssiger Phase, was im allgemeinen bevorzugt ist, sollten mindestens der Hauptteil der reagierenden Kohlenwasserstoffe flüssig vorliegen. Die flüssige Phase enthält suspendierten Kohlenstoff, der entweder während der Reaktion entsteht, zugesetzt wird oder als Bestandteil der Kohlenwasserstoffe vorhanden ist. Durch Zugabe von elementarem Kohlenstoff zum Reaktionsgemisch wird die Reaktion beschleunigt und der Umsatz gesteigert, insbesondere wenn der Kohlenwasserstoff keine oder nur geringe Mengen an Kohlenstoff enthält. Vorzugsweise liegen im Reaktionsgemisch 1 bis 50, insbesondere 5 bis 20 Gew.% Kohlenstoff vor. Geeignete Kohlenstoffzusätze sind z.B. Petrolkoks und Ruß oder eine andere Form des Graphits, besonders vorteilhaft verwendet man Aktivkohlen wie Carboraffin P® oder Tierkohle, die z.B. mit $ZnCl_2$, Phosphorsäure oder Wasserstoff aktiviert sind.

Erfindungsgemäß wird die Dehalogenierung in Gegenwart von Kohlenstoff und zusätzlich in Gegenwart einer Eisenverbindung als Cokatalysator durchgeführt. Als Eisenverbindungen kommen zwei- und/oder dreiwertige Verbindungen des Eisens in Betracht, z.B. Eisenhalogenide wie Chloride oder Bromide, Eisensulfat, Eisennitrat, Eisenphosphat, Eisenrhodanid, Eisenchromat, Eisen(II)-oxalat, Eisen(III)-acetat, Eisen(III)-formiat und insbesondere Eisenoxide, Eisenoxidhydrate oder Eisensulfide. Beispielsweise seien folgende Verbindungen aufgeführt: Eisen(II)-oxid, Eisen(III)-oxid, ($\alpha$- oder $\gamma$-Modifikation), Eisen(II,III)-oxid, Eisenoxidhydrat wie Geothit oder Lepidokrokit, Eisen(II)-sulfid, Eisen(II)-disulfid (Pyrit) oder Eisen(III)-sulfid. Es können auch Gemische der genannten Verbindungen oder elementares Eisen verwendet werden. Besonders bevorzugt sind Eisen(II)-sulfid und -disulfid sowie inbesondere $Fe_2O_3$.

Durch den Zusatz dieser Eisenverbindungen als Cokatalysator neben Kohlenstoff kann die bisher

für die Wasserstoffübertragung benötigte Kohlenwasserstoffmenge drastisch reduziert werden. Außerdem kann dann die Enthalogenierungsreaktion bereits bei niedrigeren Temperaturen mit hohen Umsätzen durchgeführt werden.

Die Menge des Eisencokatalysators im Reaktionsgemisch beträgt im allgemeinen ca. 0,001 bis 10 Gew.%, insbesondere 0,5 bis 5 Gew.%, bezogen auf Kohlenwasserstoff/Kohlenstoff. Größere Mengen sind möglich, werden aber in der Regel nicht benötigt. Man kann die Eisenverbindung dem Reaktionsgemisch zusetzen, es ist aber auch möglich, Kohlepulver oder -formkörper vorher mit den Eisenverbindungen zu tränken ggf. zu calcinieren, und dann die mit den Eisenverbindungen beladenen Aktivkohle-Kontakte einzusetzen, was sich insbesondere bei Reaktionen in der Gasphase anbietet.

Die Gasphasendehalogenierung kann in Festbett- oder Wirbelbettfahrweise durchgeführt werden, wobei der Kohlenstoff in Form von Tabletten oder Strängen oder als Wirbelkontakt, z.B. mit Kohlenstoffpartikeln unterhalb von 1 mm Durchmesser verwendet wird. Es ist auch möglich, den aktiven Kohlenstoff im Umsetzungsgemisch zu erzeugen, indem man die Umsetzung in Gegenwart einer oberflächenaktiven festen Phase durchführt, wodurch vom ersten Augenblick der Reaktion an hieran katalytisch wirksamer Kohlenstoff abgeschieden wird.

Als feste Phase werden vorteilhaft oberflächenaktive Mineralien wie Kieselgel, Aluminiumoxid usw. verwendet. Beispielsweise kommen dazu auch die Oxide der Elemente der Hauptgruppe II, III, IV und/oder V des Periodensystems sowie der Nebengruppe IV in Betracht, wie MgO, $MgSiO_3$, CaO, $B_2O_3$, $TiO_2$, insbesondere Oxide des Siliziums oder Aluminiums.

Als organische Halogenverbindungen werden solche Verbindungen verwendet, bei denen mindestens ein Halogenatom wie Iod, Brom oder Chlor kovalent am Kohlenstoff gebunden ist. Diese Ausgangsstoffe können aliphatische, cycloaliphatische, aromatische oder araliphatische Halogenverbindungen, wie sie z.B. in der DE-OS 35 10 033 oder 35 10 034 beschrieben sind, sein.

So kommen z.B. unverzweigte, verzweigte oder cyclische Monohalogenalkane wie Chlorethan, 1-Chlor-2-phenyl-ethan, 1-Chlorpropan, 1-Chlor-2-methylpropan, 2-Brompropan, 1-Iodbutan, 1-Chlorpentan, tert.-Butyliodid, Chlorcyclohexan oder -cyclopentan in Betracht.

Weiterhin können olefinisch ungesättigte Monohalogenverbindungen zu Alkenen dehalogeniert werden, z.B. Allylchlorid, 1-Brombut-1-en, 2-Brombut-2-en, 1-Iodpent-2-en, 1-Chlorcyclohex-1-en, 1-Bromcyclohex-2-en, Cinnamylchlorid oder β-Bromstyrol.

Besonders geeignete Ausgangsstoffe sind vicinale Dihalogenide der allgemeinen Formel I

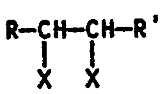 I,

in der X für Iod, Brom und/oder Chlor steht und die Reste R und R′ unabhängig voneinander Wasserstoff, einen aliphatischen Rest, z.B. eine Alkylgruppe mit 1 bis 20, insbesondere 1 bis 5 Kohlenstoffatomen, eine Cycloalkylgruppe, z.B. mit 5 bis 8 Kohlenstoffatomen, eine Arylgruppe oder eine Aralkylgruppe, insbesondere mit 6 bis 12 Kohlenstoffatomen bedeuten oder beide Reste zusammen mit den C-Atomen, an die sie gebunden sind zu einem Cycloalkan mit 4 bis 12, insbesondere 4 bis 8 Kohlenstoffatomen verbunden sind. Weiterhin können vorteilhaft Polyhalogenverbindungen umgesetzt werden.

Beispielsweise kommen Verbindungen wie 1,2-Dichlorpropan, 1,2-Dichlorbutan, 1,2-Dichlorpentan, 1,2-Dibrombutan, 2,3-Diiodbutan, 1,2-Dibrom-2-methylpropan, 1,2-Dibromcyclobutan oder -cyclohexan, 1,2-Dichlor-1,2-diphenylethan, 1,2-Dichlor-3-phenyl-propan, 1-Brom-2-iodbutan, 1-Brom-2-chlorethan in Betracht sowie 1,1,2-Trichlorethan oder Hexachlorcyclohexan (HCH).

Durch die beschriebenen Ausgangsverbindungen soll die Anwendungsbreite des erfindungsgemäßen Verfahrens nicht eingeschränkt werden. Die Halogenverbindungen können zusätzlich unter den Reaktionsbedingungen inerte Substituenten wie Cyano-, Alkoxy-, Dialkylamino-, Phenyl- oder substituierte Phenylgruppen tragen. Weiterhin können heterocyclische Verbindungen, z.B. halogensubstituiertes Pyridin, Chinolin, Pyrrol oder Imidazol umgesetzt werden.

Die Dehalogenierung von Hexachlorcyclohexan führt je nach Reaktionstemperatur zu 1,3,5-Trichlor-, m-Dichlor-, Monochlor- und schließlich Benzol.

Als aromatische Halogenverbindungen werden z.B. Halogenbenzole wie Brombenzol, meta-Dichlorbenzol, 1,3,5-Trichlorbenzol, Halogentoluole wie p-Bromtoluol, 2,4-Dichlortoluol, halogenierte Biphenyle oder Triphenyle wie Diphenyl- oder Triphenylchloride oder Polychlorbiphenyl (PCB) sowie halogenierte Naphthaline wie Chlor- oder Bromnaphthalin, Penta- oder Hexachlornaphthalin umgesetzt.

Die aromatisch gebundenen Halogenatome werden durch Wasserstoff substituiert. Mehrfach halogenierte Aromaten werden stufenweise reduktiv bis zum halogenfreien Kohlenwasserstoff enthalogeniert. So entsteht durch Umsetzung von Polychlorbiphenyl mit Vakuumrückstand Chlorwasserstoff und Diphenyl.

Sehr vorteilhaft können nach dem erfindungsgemäßen Verfahren aromatische oder heteroaromatische Acylhalogenide, insbesondere Säurebromide oder -chloride selektiv zu Aldehyden reduziert werden. Beispielsweise kommen als Ausgangsstoffe Benzoylchlorid, Salicylsäurechlorid, 1- oder 2-Naphthoylchlorid, Nicotin- oder Isonictionsäurechlorid, Picolinsäurechlorid, 2-Furoylchlorid, Thiophen- oder Pyrrol-2-carbonsäurechlorid bzw. die entsprechenden Säurebromide in Betracht. Die genannten Reste können noch unter den Reaktionsbedingungen inerte Substituenten tragen, z.B. Halogen-, Alkyl-, Alkoxy-, Hydroxyl-, Nitril-, Ester-, Säure- oder Aminogruppen.

Nach dem erfindungsgemäßen Verfahren lassen sich auch Dicarbonsäurehalogenide reduzieren, wobei jedoch teilweise Überhydrierung oder Ringschlußreaktionen eintreten können.

Die erfindungsgemäße Umsetzung kann diskontinuierlich oder kontinuierlich bei Normaldruck, erhöhtem oder vermindertem Druck nach den dafür üblichen Techniken, z.B. in einem Rührreaktor oder in einem zylindrischen Reaktor mit Umlauf durchgeführt werden.

Zweckmäßigerweise verfährt man so, daß man die Halogenverbindungen fest, flüssig oder gasförmig, gegebenenfalls zusammen mit einem Inertgas, z.B. Stickstoff, dem auf Reaktionstemperatur erhitzten Reaktor zuführt. Nach der Umsetzung verlassen die Reaktionsprodukte in der Regel gasförmig zusammen mit dem gebildeten Halogenwasserstoff den Reaktor. Die Produkte werden isoliert, indem man sie z.B. je nach Siedepunkt entweder vor oder nach Abtrennung des Halogenwasserstoffs kondensiert und gegebenenfalls z.B. destillativ reinigt. Die Abtrennung der Halogenwasserstoffsäuren erfolgt zweckmäßig durch eine Wasserwäsche. Die so erhaltenen Halogenwasserstoffsäuren werden dann neutralisiert und einer Nutzung zugeführt. Gegebenenfalls kann auch der Halogenwasserstoff unmittelbar durch eine Laugenwäsche neutralisiert werden.

Die nachfolgenden Beispiele erläutern die Erfindung und zeigen die gegenüber der in der DE-OS 35 10 033 beschriebene Verfahrensweise erzielte Verbesserung bei Verwendung des Eisencokatalysators.

Beispiel 1a

Dehalogenierung von 1,2-Dichlorpropan

In einem 2-l-Rührkolben wurden 900 g Technisches Weißöl, 100 g Aktivkohle und 5 g Eisenoxid ($Fe_2O_3$) gemischt und auf Reaktionstemperatur von 350°C erhitzt. Stündlich wurden ca. 57 g (0,5 mol) 1,2-Dichlorpropan unter gleichzeitigem Einleiten von ca. 3 l Stickstoff pro Stunde unter der Oberfläche des gerührten Reaktionsgemisches zugeführt. Der gasförmige Reaktionsaustrag wurde zunächst bei Temperaturen von -10 bis 0°C kondensiert, um nichtumgesetztes 1,2-Dichlorpropan sowie leichtsiedende Bestandteile aus dem Öl abzutrennen. Anschließend wurde der Gasstrom zwecks Absorption des gebildeten Chlorwasserstoffs durch eine Glockenbodenkolonne mit Wasser geleitet und so eine wäßrige Salzsäure gewonnen. Das die Glockenbodenkolonne verlassende Abgas, hauptsächlich Propen, wurde über eine Gasuhr erfaßt und gaschromatographisch untersucht. Die erhaltene Salzsäure wurde durch Titration bestimmt. Die Aktivität dieses Reduziersystems, d.h. der Umsatz an 1,2-Dichlorpropan, nahm dabei erst nach 40 Stunden Versuchsdauer deutlich ab.

Insgesamt wurden bei einer Versuchsdauer von 46 Stunden 2610 g (23,1 mol) 1,2-Dichlorpropan eingeleitet, wovon 441 g (3,9 mol) als nicht umgesetzt zurückgewonnen wurden. Dabei wurden 738 g (17,5 mol) Propen und 1345 g (36,9 mol) Chlorwasserstoff erzeugt. Dies entspricht über die gesamte Versuchsdauer einem durchschnittlichen Umsatz von 83,1 %, wobei die Propen-Selektivität 91,1 % beträgt.

Beispiel 1b

Dehalogenierung von 1,2-Dichlorpropan (Vergleichsbeispiel)

900 g Technisches Weißöl wurden mit 100 g Aktivkohle gemischt und auf Reaktionstemperatur von 350°C erhitzt. Desweiteren wurde wie in Beispiel 1a beschrieben verfahren. Die Aktivität dieses Reduziersystems (ohne Anwesenheit des Eisencokatalysators) fiel jedoch bereits nach relativ kurzer Versuchsdauer stark ab: Während anfangs pro Stunde 14,7 g (0,35 mol) Propen erzeugt wurden, betrug dieser Wert nach 6 Stunden nur noch 5,0 g (0,12 mol). Über die gesamte Versuchsdauer von 8 Stunden konnten damit lediglich 75 g (1,8 mol) Propen hergestellt werden.

Beispiel 2

Dehalogenierung von 1,2-Dichlorpropan

Die Dehalogenierung von 1,2-Dichlorpropan wurde wie in Beispiel 1a beschrieben durchgefürt, doch wurden anstelle von 5 g Eisenoxid, 3,9 g Eisensulfid (FeS) als Cokatalysator zugesetzt. Bei einer Versuchsdauer von 44 Stunden wurden insgesamt 2520 g (22,3 mol) 1,2-Dichlorpropan eingeleitet, wovon 496 g (4,4 mol) nicht umgesetzt wurden. Dabei wurden 676 g (16,1 mol) Propen und 1244 g (34,1 mol) Chlorwasserstoff erzeugt. Dies entspricht über die gesamte Versuchsdauer einem durchschnittlichen Umsatz von 80,3 %, wobei die Propen-Selektivität 89,9 % beträgt.

Beispiel 3a

Dehalogenierung von 1,2-Dichlorpropan

Es wurde wie in Beispiel 1a verfahren, doch betrug die Reaktionstemperatur lediglich 300°C. Dabei wurden stündlich neben 12,4 g (0,11 mol) nicht umgesetztem 1,2-Dichlorpropan 14,3 g (0,34 mol) Propen und 27,0 g (0,74 mol) Chlorwasserstoff erhalten. Dies entspricht einem Umsatz von 78,2 % und einer Propen-Selektivität von 87,2 %.

Beispiel 3b

Dehalogenierung von 1,2-Dichlorpropan (Vergleichsbeispiel)

Es wurde wie in Beispiel 3a verfahren, doch wurde dem Reaktionsgemisch kein Eisenoxid zugesetzt. Dabei wurden stündlich neben 37 g (0,33 mol) nicht umgesetztem 1,2-Dichlorpropan 6,4 g (0,15 mol) Propen erhalten. Dies entspricht einem Umsatz von lediglich 35,1 % bei einer Propen-Selektivität von 88,2 %.

Beispiel 4a

Dehalogenierung von Brombenzol

In der in Beispiel 1a beschriebenen Apparatur wurden 900 g Technisches Weißöl, 100 g Aktivkohle und 5 g Eisenoxid ($Fe_2O_3$) vorgelegt und auf Reaktionstemperatur von 350°C erhitzt. Stündlich wurden ca. 79 g (0,5 mol) Brombenzol unter gleichzeitigem Einleiten von ca. 3 l Stickstoff pro Stunde unter der Oberfläche des gerührten Reaktionsgemisches zugeführt. Bei einer Versuchsdauer von vier Stunden wurden 296 g Kondensat erhalten, das aufgrund einer gaschromatographischen Analyse zu 79,7 Gew.%, entsprechend 235,9 g, aus Brombenzol und 12,5 Gew.%, entsprechend 37,0 g, aus Benzol bestand. Der Umsatz betrug demnach 25,3 % und die Selektivität 93,1 %.

Beispiel 4b

Dehalogenierung von Brombenzol (Vergleichsbeispiel)

Es wurde wie in Beispiel 4a verfahren, doch wurde dem Reaktionsgemisch kein Eisenoxid zugesetzt. Bei einer Versuchsdauer von vier Stunden wurden 331 g Kondensat erhalten, das aufgrund einer gaschromatographische Analyse zu 91,2 Gew.%, entsprechend 301,9 g, aus Brombenzol und 1,9 Gew.%, entsprechend 6,3 g, aus Benzol bestand. Der Umsatz betrug demnach nur 4,5 % bei einer Selektivität von 89,2 %.

Beispiel 5a

Dehalogenierung von 1-Chlorpropan

In der im Beispiel 1a beschriebenen Apparatur wurden 900 g Technisches Weißöl, 100 g Aktivkohle und 5 g Eisenoxid ($Fe_2O_3$) vorgelegt und auf Reaktionstemperatur von 350°C erhitzt. Stündlich wurden ca. 39 g (0,5 mol) 1-Chlorpropan unter gleichzeitigem Einleiten von 1 bis 2 l Stickstoff pro Stunde unter der Oberfläche des gerührten Reaktionsgemisches zugeführt. Desweiteren wurde wie in Beispiel 1a verfahren. Stündlich wurden ca. 21 g (0,27 mol) nicht umgesetztes 1-Chlorpropan zurückgewonnen und 2,9 g (0,07 mol) Propen sowie 6,2 g (0,14 mol) Propan erzeugt. Dies entspricht einem Umsatz von 46,0 % und einer Propan-Selektivität von 60,9 %.

Beispiel 5b

Dehalogenierung von 1-Chlorpropan (Vergleichsbeispiel)

Es wurde wie in Beispiel 5a verfahren, doch wurde dem Reaktionsgemisch kein Eisenoxid zugesetzt. Stündlich wurden ca. 33 g (0,42 mol) nicht umgesetztes 1-Chlorpropan zurückgewonnen und 1,7 g (0,04 mol) Propen sowie 1,3 g (0,03 mol) Propan erzeugt. Dies entspricht einem Umsatz von 16,0 % und einer Propan-Selektivität von 37,5 %.

Beispiel 6a

Dehalogenierung von 1,2-Dichlorbenzol

Stündlich wurden ca. 15 g (0,1 mol) 1,2-Dichlorbenzol und ca. 26 g (0,2 mol) Tetralin bei 275°C vorverdampft und zusammen mit ca. 2 l Stickstoff pro Stunde einem auf 400°C beheizten Festbett-Reaktor zugeführt, der mit 100 g eines mit 7,2 Gew.% $Fe_2O_3$-belegten Supersorbon®-Katalysators gefüllt war. Die gasförmigen Reaktionsprodukte wurden zunächst bei -10°C kondensiert und der Gasstrom anschließend zwecks Entfernung des gebildeten Chlorwasserstoffs durch eine Glockenbodenkolonne mit Wasser geleitet. Eine Titration der gebildeten Salzsäure ergab, daß pro Stunde 5,5 g (0,15 mol) Chlorwasserstoff erzeugt wurden. Demnach wurden 75 % des im 1,2-Dichlorbenzol gebundenen Chlors reduktiv entfernt. Im Kondensat wurden als Reaktionsprodukte Benzol und Chlorbenzol nachgewiesen.

Beispiel 6b

Dehalogenierung von 1,2-Dichlorbenzol (Vergleichsbeispiel)

Es wurde wie in Beispiel 6a verfahren, doch wurde als Katalysator Supersorbon® ohne Metall-Zusatz verwendet. Die Titration der gebildeten Salzsäure ergab, daß pro Stunde 4,0 g (0,11 mol) Chlorwasserstoff erzeugt wurden. Demnach wurden nur 55 % des im 1,2-Dichlorbenzol gebundenen Chlors reduktiv entfernt.

**Patentansprüche**

1. Verfahren zur reduktiven Dehalogenierung von organischen Halogenverbindungen, bei denen mindestens ein Halogenatom kovalent am Kohlenstoff gebunden ist, durch Umsetzung mit Kohlenwasserstoffen in Gegenwart von Kohlenstoff bei erhöhter Temperatur unter Bildung von Halogenwasserstoffen, dadurch gekennzeichnet, daß man die Dehalogenierung in Gegenwart von Eisenpulver oder einer Eisenverbindung als Cokatalysator bei Temperaturen von 100 bis 450°C durchführt.

2. Verfahren gemäß Anspruch 1, dadurch gekennzeichnet, daß man als Kohlenwasserstoffe hochsiedende Mineralöle verwendet, deren Siedepunkte höher liegen als die Reaktionstemperatur.

3. Verfahren gemäß den Ansprüchen 1 und 2, dadurch gekennzeichnet, daß man als Kohlenwasserstoff Vakuumrückstand, Heizöl S oder Technisches Weißöl verwendet.

4. Verfahren gemäß den Ansprüchen 1 bis 3, dadurch gekennzeichnet, daß man als Eisenverbindung Eisen(III)-oxid, Eisen(II)-sulfid oder Eisen(II)-disulfid einsetzt.

5. Verfahren gemäß den Ansprüchen 1 bis 4, dadurch gekennzeichnet, daß im Reaktionsgemisch 1 bis 50 Gew.% Kohlenstoff sowie 0,1 bis 10 Gew.% der Eisenverbindung vorliegen.

6. Verfahren gemäß Anspruch 1, dadurch gekennzeichnet, daß man niedrig siedende Kohlenwasserstoffe oder deren Gemische wie Heizöl L, Benzin, Leichtbenzin oder Flüssiggase verwendet und die Umsetzung entweder unter erhöhtem Druck in der Flüssigphase oder drucklos in der Gasphase durchführt.

7. Verfahren gemäß Anspruch 1, dadurch gekennzeichnet, daß man olefinisch ungesättigte Monohalogenverbindungen umsetzt.

8. Verfahren gemäß Anspruch 1, dadurch gekennzeichnet, daß man vicinale Di- oder Polyhalogenalkane bzw. -Cycloalkane umsetzt.

9. Verfahren gemäß Anspruch 1, dadurch gekennzeichnet, daß man aromatische Halogenverbindungen umsetzt.

10. Verfahren gemäß Anspruch 1, dadurch gekennzeichnet, daß man aromatische oder heteroaromatische Acylhalogenide umsetzt.

## Claims

1. A process for the reductive dehalogenation of an organic halogen compound in which one or more halogen atoms are covalently bonded to the carbon, by reaction with a hydrocarbon in the presence of carbon at elevated temperatures with formation of a hydrogen halide, wherein the dehalogenation is carried out in the presence of iron powder or an iron compound as a cocatalyst, at from 100 to 450°C.

2. A process as claimed in claim 1, wherein the hydrocarbon used is a high boiling mineral oil whose boiling point is higher than the reaction temperature.

3. A process as claimed in claims 1 and 2, wherein the hydrocarbon used is a vacuum residue, heavy fuel oil or industrial white oil.

4. A process as claimed in any of claims 1 to 3, wherein the iron compound used is iron(III) oxide, iron(II) sulfide or iron(II) disulfide.

5. A process as claimed in any of claims 1 to 4, wherein from 1 to 50% by weight of carbon and from 0.1 to 10% by weight of the iron compound are present in the reaction mixture.

6. A process as claimed in claim 1, wherein a low boiling hydrocarbon or a mixture or such hydrocarbons, such as light fuel oil, gasoline, naphtha or liquefied petroleum gas, is used, and the reaction is carried out either under superatmospheric pressure in the liquid phase or under atmospheric pressure in the gas phase.

7. A process as claimed in claim 1, wherein an olefinically unsaturated monohalogen compound is reacted.

8. A process as claimed in claim 1, wherein a vicinal di- of polyhaloalkane or -cycloalkane is reacted.

9. A process as claimed in claim 1, wherein an aromatic halogen compound is reacted.

10. A process as claimed in claim 1, wherein an aromatic or heteroaromatic acyl halide is reacted.

## Revendications

1. Procédé de déshalogénation réductrice de composés halogénés organiques dans lesquels au moins un atome d'halogène est fixé par liaison covalente sur le carbone, par réaction avec des hydrocarbures en présence de carbone à température élevée avec formation d'acide halogénhydrique, caractérisé en ce qu'on conduit la déshalogénation à des températures de 100 à 450°C en présence de poudre de fer ou d'un composé du fer servant de co-catalyseur.

2. Procédé selon la revendication 1, caractérisé en ce qu'on utilise, comme hydrocarbures, des huiles minérales de point d'ébullition élevé, dont les températures d'ébullition se situent au-dessus de la température de réaction.

3. Procédé selon la revendication 1 ou 2, caractérisé en ce qu'on utilise, comme hydrocarbure, un résidu court de la distillation poussée, une huile lourde ou une huile blanche technique.

4. Procédé selon l'une quelconque des revendications 1 à 3, caractérisé en ce qu'on utilise, comme composé du fer, de l'oxyde de fer (III), du sulfure de fer (II) ou du disulfure de fer (II).

5. Procédé selon l'une quelconque des revendications 1 à 4, caractérisé en ce que 1 à 50% en poids de carbone et 0,1 à 10% en poids du composé du fer sont présents dans le mélange réactionnel.

6. Procédé selon la revendication 1, caractérisé en ce qu'on utilise des hydrocarbures de bas point d'ébullition ou leurs mélanges, tels que du fioul L, de l'essence, de l'essence minérale légère ou des gaz combustibles liquéfiés, et en ce que la réaction est conduite, soit sous pression élevée en phase liquide, soit sans pression en phase gazeuse.

7. Procédé selon la revendication 1, caractérisé en ce qu'on fait réagir des composés monohalogénés à insaturation oléfinique.

8. Procédé selon la revendication 1, caractérisé en ce qu'on fait réagir des di- ou polyhalogéno-alcanes ou -cycloalcanes vicinaux.

9. Procédé selon la revendication 1, caractérisé en ce qu'on fait réagir des composés halogénés aromatiques.

10. Procédé selon la revendication 1, caractérisé en ce qu'on fait réagir des halogénures d'acyle aromatiques ou hétéroaromatiques.